(19) Europäisches Patentamt
European Patent Office
Office européen des brevets

(11) Publication number: **0 068 680**
**B1**

(12) **EUROPEAN PATENT SPECIFICATION**

(45) Date of publication of patent specification: **17.09.86**

(21) Application number: **82302977.2**

(22) Date of filing: **09.06.82**

(51) Int. Cl.⁴: **C 07 D 407/06,** C 12 P 17/16, A 61 K 31/35 // (C12P17/16, C12R1:39)

(54) **Antibiotics.**

(30) Priority: **20.06.81 GB 8119111**

(43) Date of publication of application:
**05.01.83 Bulletin 83/01**

(45) Publication of the grant of the patent:
**17.09.86 Bulletin 86/38**

(84) Designated Contracting States:
**CH DE FR GB IT LI NL**

(56) References cited:
**GB-A-1 587 059**
**GB-A-2 005 680**

(73) Proprietor: **BEECHAM GROUP PLC**
**Beecham House Great West Road**
**Brentford Middlesex TW8 9BD (GB)**

(72) Inventor: **O'Hanlon, Peter John**
**65 Green Lane**
**Redhill Surrey (GB)**

(74) Representative: **Cresswell, Thomas Anthony et al**
**European Patent Attorney**
**Beecham Pharmaceuticals**
**Great Burgh Yew Tree Bottom Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 068 680 B1

**Description**

The present invention relates to novel antibiotics having activity against bacteria and mycloplasma.

U. K. Patent No. 1,395,907 describes novel antibiotics produced by, and isolated from cultures of *Pseudomonas fluorescens* (NCIB 10585). The microorganism was deposited on 6.3.72 and is freely available to the public. Two of these, now known as "pseudomonic acid A" and "pseudomonic acid B" have the structure (I):

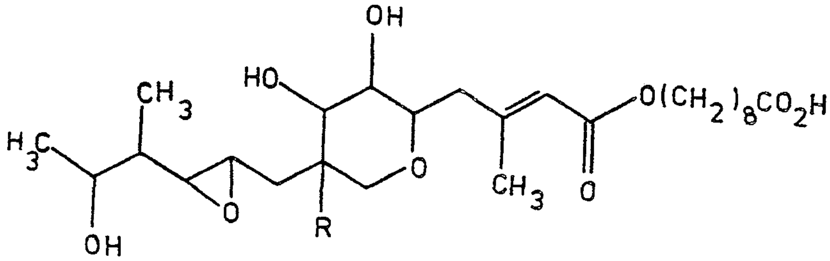

(I): R=H, "pseudomonic acid A

R=OH, "pseudomonic acid B

European Patent Application No. 0 003 069 describes a further antibiotic produced by *P. fluorescens,* known as ""pseudomonic acid C" having a structure (1A):

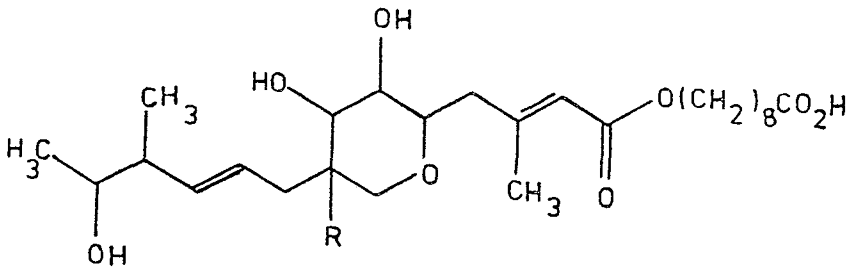

(1A): R=H, "pseudomonic acid C

U. K. Patent No. 1,587,059 describes a related compound, "monic acid A", of structure (II):

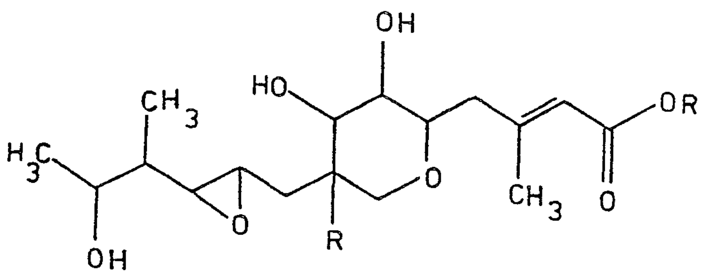

(II): R=H, monic acid A

This patent also describes esters of monic acid A (i.e., compounds of structure (II) wherein R is an ester-forming radical), including esters wherein R is a $C_{2-8}$ alkenyl group optionally substituted, *inter alia* with a carboxy group, which have antibacterial activity. It has now been found that a compound falling within the generic description of such esters, but not specifically disclosed in U. K. 1,587,059 has particular advantages not shared by the other compounds wherein R is a $C_{2-8}$ alkenyl group.

According to the present invention there is provided a process for producing "pseudomonic acid D of formula (III)

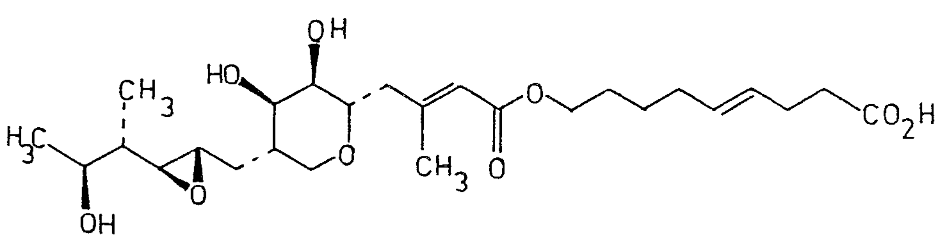

or a salt or ester thereof, which process comprises culturing an organism of *Pseudomonas fluorescens* capable of producing Pseudomonic acid D, recovering from the culture medium pseudomonic acid D of formula (III) and thereafter if desired forming a salt or ester thereof.

The compound of formula (III) has been named "pseudomonic acid D". It is known that the compound has the absolute stereochemistry shown in formula (III) above.

Salts and esters of pseudomonic acid D may be pharmaceutically acceptable but this is not essential. Non-pharmaceutically acceptable salts and esters may be useful in separation and purification of pseudomonic acid D.

Pharmaceutically acceptable salts include metal salts, (e.g. aluminium, alkali metal salts such as sodium or potassium, and alkaline earth metal salts such as calcium or magnesium) and ammonium or substituted ammonium salts (e.g. those with lower alkylamines such as triethylamine, hydroxy-lower alkylamines such as 2-hydroxyethylamine, bis-(2-hydroxyethyl)-amine or tri-(2-hydroxyethyl)-amine, cycloakylamines such as bicyclohexylamine, or with procaine, dibenzylamine, N, N-dibenzykethylene-diamine, 1-ephenamine, N-ethyl-piperidine, N-benzyl-β-phenethyl-amine, dehydroabietyl-amine, N, N'-bis-dehydroabietylethylene-diamine, or bases of the pyridine type such as pyridine, collidine or quinoline). Preferred salts are alkali metal salts.

Suitable esters are $C_{1-6}$ alkyl esters.

The preferred ester is the methyl ester.

Pseudomonic acid D is particularly advantageous in that it is produced by, and may be isolated from cultures of *P. fluorescens* (NCIB 10586). The other compounds disclosed in U. K. 1,587,059 are, of course, all semi-synthetic antibiotics.

Pseudomonic acid D, its salts and esters are preferably in crystalline form.

Salts and esters of Pseudomonic acid D may be produced by conventional methods.

The process of the invention is suitably carried out under conventional fermentation conditions, for instance as shown below in the Examples. The culture medium is then acidified and the pseudomonic acids extracted by repeated solvent extractions. The crude pseudomonic acids are then separated by hplc to afford pseudomonic acid D. The details of the process are given in the Examples below.

A preferred organism of *Pseudomonas fluorescens* is NCIB 10586.

As well as being useful as an antibiotic in its own right, pseudomonic acid D is useful as an intermediate in producing other antibiotics. Thus conventional hydrogenation of pseudomonic acid D affords pseudomonic acid A (I, R=H) and monic acid A, may be obtained by hydrolysis of pseudomonic acid D, for instance by the methods described in U. K. Patent No. 1,587,058.

Accordingly, the process of the present invention may comprise a further step for producing pseudomonic acid A, which step comprises hydrogenating pseudomonic acid D.

In addition the process of the present invention may comprise a further step for producing monic acid A, which step comprises hydrolysing pseudomonic acid D.

o Pseudomonic acid D is an active antibiotic and may be used in human and vetinary treatment.

Accordingly, there may be prepared a pharmaceutical composition comprising pseudomonic acid D or a pharmaceutically acceptable salt or ester thereof in association with a pharmaceutically acceptable carrier therefor.

Tablets and capsules for oral administration may be in unit dose presentation form, and may contain conventional excipients such as binding agents, for example syrup, acacia, gelatin, sorbitol, tragacanth, or polyvinyl-pyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl *p*-hydroxybenzonoate or sorbic acid, and if desired conventional flavouring or colouring agents.

Suppositories will contain conventional suppository bases, e.g. cocoa-butters or other glyceride.

For parenteral administration, fluid unit dosage forms are prepared utilizing the compound and a sterile vehicle. The compound, depending on the vehicle and concentration used, can either be suspended or dissolved in the vehicle. In preparing solutions the compound can be dissolved in water for injection and filter sterilized before filling into a suitable vial or ampoule and sealing. Advantageously, adjuvants such as a local anaesthetic, preservative and buffering agents can be dissolved in the vehicle. To enhance the stability the composition can be frozen after filling into the vial and water removed under vacuum. The dry lypophilized powder is then sealed in the vial. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in

the composition to facilitate uniform distribution of the compounds.

The compositions may contain from 0.1% to 99% by weight, preferably from 10 to 60% by weight, of the active material, depending on the method of administration. Where the compositions comprises dosage units, each unit will preferably contain from 50—500 mg, of the active ingredient. The dosage as employed for adult human treatment will preferably range from 100 mg to 3 g, per day, for instance 250 mg to 2 g per day, depending on the route and frequency of administration.

Alternatively pseudomonic acid D or a pharmaceutically acceptable salt or ester may be administered as part of the total dietery intake. In this case the amount of compound employed may be less than 1% by weight of the diet and it preferably no more than 0.5% by weight. The diet for animals may consist of normal feedstuffs to which the compound may be added or it may be added to a premix.

A suitable method for administration pseudomonic acid D or a pharmaceutically acceptable salt or ester thereof to animals is to include it in the drinking water provided for the animals. In this case the concentration of the compound in the drinking water would be from about 5 to 500 µg/ml, for example 5 to 200 µg/ml.

Typically, pseudomonic acid D or a pharmaceutically acceptable salt or ester thereof would be administered at 1 mg/kg body weight per day to 50 mg/kg, especially 2.5 mg/kg to 25 mg/kg when given internally.

The invention will now be illustrated with reference to the following Examples, which are not intended to limit the invention in any way.

## Example 1

Production of Pseudomonic Acid D by Fermentation

*Pseudomonas fluorescens,* Strain NCIB 10586 was cultured on an agar slope and flooded with sterile water. A sample was added to the following seed stage medium:

| | |
|---|---|
| Oxoid yeast extract: | 2 (%w/v) |
| Glucose: | 0.11 |
| Disodium hydrogen orthophosphate: | 0.26 |
| Potassium dihydrogen orthophosphate: | 0.24 |

This was incubated at 28°C overnight and then used to innoculate the following production stage medium:

| | |
|---|---|
| Corn steep liquor: | 0.3 (%w/v) |
| Glucose: | 2.0 |

tGlycerol:
0.5

| | |
|---|---|
| Ammonium sulphate: | 0.2 |
| Calcium carbonate: | 0.4 |
| Potassium dihydrogen orthophosphate: | 0.04 |
| Disodium hydrogen orthophosphate: | 0.065 |
| Manganese chloride 4 $H_2O$: | 0.0003 |
| Potassium chloride: | 0.05 |
| Magnesium sulphate 7 $H_2O$: | 0.0375 |
| P2000 to minimise foaming | |

The fermentation was carried out at 25°C for 48 hours.

(b) Isolation of Pseudomonic Acid D from Culture Medium

Culture medium (1500 l) was partially clarified on a rotary pre-coat filter and further clarified by

centrifugation giving clarified liquor (1285 l). This was acidified to pH 4.5 using hydrochloric acid (50%) and was extracted with methyl isobutyl ketone (MIBK; 300 l) by metering the liquor at 8 1 $min^{-1}$ and solvant at 2 1 $min^{-1}$ into an in-line static mixer.

The two phases were separated by centrifugation and the organic extract was extracted with sodium bicarbonate solution (2% w/v, 60 l) pre-adjusted to pH 3.2. The phases were separated by centrifugation. MIBK (12 l) was added to the aqueous extract and the aqueous phase was acidified to pH 4.5 using hydrochloric acid (50%). The organic phase was separated, washed with deionised water (6 l), and then concentrated to 1.5 l. The concentrate was dried with magnesium sulphate. The magnesium sulphate was removed by filtration and n-heptane (760 ml) added to the dry solution. The mixture so produced was left to stand at 5°C for 12 hours. The product was filtered and washed with MIBK/heptane (50:50; 1 l) and then with heptane (1 l) and dried *in vaco* at 25°C. This material (125 g was shown by hplc analysis to contain 92—93% of pseudomonic acid A and ~2% of pseudomonic acid D.

The crude product so produced was dissolved in dry tetrahydrofuran (THF; 1.5 ml) and chromatographed in portions (200—250 μl) by high performance liquid chromography (hplc) using an Altex ultrasphere-octyl (250 mm × 10 mm) reverse column and eluting at a flow rate of 3 ml/min of ammonium acetate buffer (0.1M; pH 5.7): THF (3:1). Pseudomonic acid D had a retention time of 30 minutes (pseudomonic acid A $R_t$=41 mins). The combined eluents containing the pseudomonic acid D peaks (detected by UV at $\lambda_{max}$ 240 nm) were evaporated to dryness, then the residue dissolved in water and adjusted to pH 4 using hydrochloric acid (1M), under a layer of ethyl acetate (20 ml). The organic phase was separated and the aqueous phase further extracted with ethyl acetate, (3 × 20 ml), then the combined extracts washed with brine, dried with magnesium sulphate and evaporated to dryness. The pseudomonic acid D obtained was rechromatographed by hplc as above and the combined eleuents evaporated to remove the THF, then the aqueous solution was adjusted to pH 7 and washed with ethyl acetate. The aqueous layer was acidified to pH 4 using hydrochloric (1M) under a layer of ethyl acetate (25 ml) and the aqueous layer further extracted with ethyl acetate (3 × 25 ml). The combined extracts were dried using magnesium sulphate and evaporated to yield pseudomonic acid D (30 mg).

ir spectrum

$\nu_{max}$ ($CHC1_3$) 3400, 1720 and 1650 $cm^{-1}$;

U. V. spectrum

$\lambda_{max}$ (EtOH) 225 nm;

$^1H$ nmr δH ($CDC1_3$)

5.76 (1H, m, H−2), 5.45 (2H, m, CH=CH, J=15Hz), 4.08 (3H, t, $CH_2$−9′), 3.91 (1H, dd, H−7), 3.87 and 3.54 (2H, m, $CH_2$−16), 3.80 (1H, m, H−13), 3.76 (1H, m, H−5), 3.47 (1H, dd, H−6), 2.83 (1H, dt, H−10), 2.73 (1H, dd, H−11), 2.59 and 2.30 (2H, m, $CH_2$−4), 2.40 (2H, t, $CH_2$−2′), 2.32 (2H, m, $CH_2$−3′), 2.21 (3H, d, $CH_3$−15), 2.02 (2H, m, $CH_2$−6′), 2.00 (1H, m, H−8), 1.73 (2H, m, $CH_2$−9), 1.63 (2H, m, $CH_2$−8′), 1.44 (2H, m, $CH_2$−7′), 1.38 (1H, m, H−12), 1.22 (3H, d, $CH_3$−14), 0.94 (3H, d, $CH_3$−17).

$^{13}C$ nmr δC ($CDC1_3$)

178.0 (C1′), 166.9 (C1), 156.6 (C3), 131.4 (C5′), 128.5 (C4′), 117.8 (C2), 75.2 (C5), 71.4 (C13), 70.6 (C7), 69.2 (C6), 65.5 (C16), 63.8 (C9′), 61.3 (C11), 55.7 (C10), 42.9 (C4, 12) 39.6 (C8), 34.2 (C2′), 32.0, 21.8, 28.1, 27.8, 25.9 (C6′, 9, 8′, 7′, 3′), 20.8 (C17), 19.3 (C15), 12.7 (C17).

Mass spectrum m/e (relative intensity) 498 ($M^+$, 3%) 396 (4), 327 (10), 309 (16), 291 (5), 254 (23), 227 (92), 209 (30), 141 (49), 137 (39), 129 (22), 111 (100), (Found: 498.2860. $C_{26}H_{42}O_9$ requires 498.2892) (Found: 254.1505. $C_{14}H_{22}O_4$ requires 254.1944).

Structure Elucidation of Pseudomonic Acid D isolated from NCIB 10586

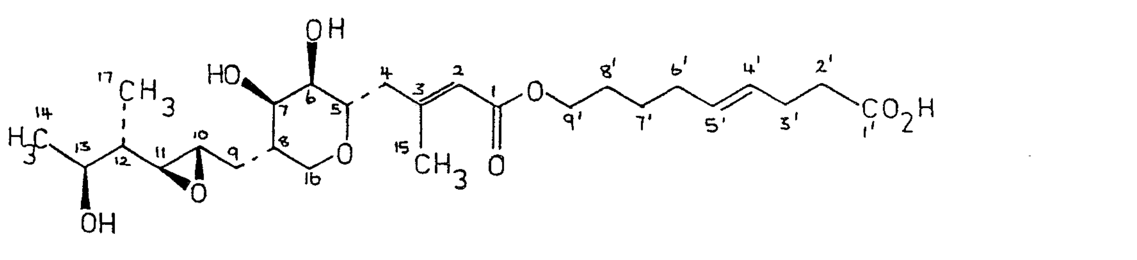

Purified pseudomonic acid D was shown by high resolution mass spectroscopy to have a molecular weight of of 498.2860 indicating a molecular formula of $C_{26}H_{42}O_9$, which is two hydrogen atoms less than that of pseudomonic acid A. The infra red spectrum had absoptions at $\nu_{max}$ 1720 (C=O) and 1650 (C=C) $cm^{-1}$ indicating the compound to be an unsaturated ester, which was confirmed by the u.v. absorption at $\lambda_{max}$ 225 nm, thus showing the structural similarity of pseudomonic acid D to the other three naturally occurring metabolites, pseudomonic acids A, B and C. The $^{13}C$ and $^1H$ nmr spectra of pseudomonic acids A and D also indicated structural similarities.

The $^{13}C$ nmr spectrum of pseudomonic acid D contained all of the resonances associated with the epoxide-containing nucleus of pseudomonic acid A, i.e. carbons 1—17. However, in addition to the usual low field carbon resonances of C1, C2, C3 and C1′, two further resonances at $\delta_C$ 131.4 and 128.4, which

appeared as doublets in the off resonance spectrum, indicated the presence of a disubstituted carbon, carbon double bond in a nine carbon ester side chain. The presence of six methylene triplets in the off resonance spectrum also confirmed the absence of branching in the nonenomic acid side chain. The location of the double bond and the assignment of its geometry were determined by high resolution 250 MHz $^1$Hnmr spectroscopy.

The most notable feature of the $^1$H nmr spectrum in comparison with that of pseudomonic acid A was the presence of signals due to two olefinic protons appearing as a complex multiplet centred at δ 5.45 in deuteriochloroform. After addition of deuteriated water to exchange the hydroxyl protons and thus sharpen the spectrum, a series of spin-spin decoupling experiments were carried out. The initial studies confirmed the integrity of the C1—17 nucleus with assignments of the respective proton signals. The remaining protons unassigned were those of the nonenoic acid side chain and consisted of a multiplet at δ 5.45 for the two olefinic protons, a two proton triplet at δ 4.08 and five two-proton multiplets at δ 2.40, 2.32, 2.02, 1.63 and 1.44.

The triplet at δ 4.08 was assigned to the $CH_2$—9′ adjacent to the acrylic ester function. Further assignments are based on the following experimental observations:

The triplet at δ 4.08 was assigned to the the $CH_2$—9′ adjacent to the acrylic ester function. Further assignments are based on the following experimental observations:

(a) Irradiation of the triplet δ 4.08 produced a considerable simplification of the multiplet at δ 1.63. This signal then showed 2nd order coupling and was perturbed to high field. Irradiation at δ 1.63 collapsed this triplet to a broad singlet.

(b) Irradiation of the olefinic multiplet at δ 5.45 produced simplification of the multiplets at δ 2.32 and 2.02. The resonance at δ 2.32 then had 2nd order characteristics and was very strongly perturbed to low field, whilst that at δ 2.02 became discernable triplet even though H—8 was superimposed.

(c) Irradiation at δ 2.02 simplified the low field half of the olefinic region and collapsed the multiplet at δ 1.44 to 2nd order appearance perturbed to low field.

(d) Irradiation at δ 2.32 simplified the high field half of the olefinic region but had no effect on any of the observable methylenes. The resonance at δ 2.40 was too close to observe. The resonance at δ 2.40 had 2nd order characteristics and was perturbed very strongly to high field. Irradiation at δ 2.40 had no effect on the observable methylenes. Perturbation and appearance implied that these two methylene signals are coupled to each other. A chemical shift of δ 2.40 is in close agrrement with that of the $CH_2$ adjacent to the acid carboxyl in pseudomonic acid A.

The evidence of chemical shift and response to irradiations, as well as the implications arrived at from perturbations and 2nd order characteristics lead to the proposed construction of the side chain shown below:

| | 9′ | 8′ | 7′ | 6′ | 5′ | 4′ | 3′ | 2′ | 1′ |
|---|---|---|---|---|---|---|---|---|---|
| —CO(=O)— | $CH_2$— | $CH_2$— | $CH_2$— | $CH_2$— | CH= | CH— | $CH_2$— | $CH_2$— | $CO_2H$ |
| | 4.08 | 1.63 | 1.44 | 2.02 | 5.48 | 5.42 | 2.32 | 2.40 | |

Comfirmation of the structure of the side chain was provided by using the Inter Nucleur Double Resonance (INDOR) technique. The method clearly established the connectivity of four methylenes at C6′—C9′, when initially observing the resonance at δ 4.08 assigned to $CH_2$—9. The double resonance experiments showed clearly that the resonance at δ 2.02 assigned to the CH—6′ was coupled to the low field olefinic proton at δ 5.48. Thus the double bond is located between C4′ and C5′.

The stereochemistry of the double bond was confirmed as *trans* from the coupling constant $J_{4',5'}=15\pm0.2$ Hz, which was measured after irradiation of $CH_2$—3′ at δ2.32 and also $CH_2$6′ at δ 2.02. The assignment of the *trans* geomentry was confirmed by computer simulation using the Brucker PANIC 9081 computer programme, (Parameter Adjustment in NMR by Interaction Calculation). When programmed with the chemical shifts, coupling constants, linewidths etc., measured from the spectrum, the computer simulated an almost identical spectral pattern to that which is obtained experimentally. Using the same parameters but with a coupling constant J=9.11 Hz (appropriate for a *cis* geometry, the simulated spin-system was not comparable with that observed experimentally.

The structure of pseudomonic acid D is, therefore, confirmed with *trans* 4′ double bond present in the ester side chain.

Example 2

Sodium pseudomonate D

Methyl pseudomonate D (0.136 g) was dissolved in N,N-dimethylformamide (4.5 ml) and diluted with phosphate buffer (0.05M, pH 7, 60 ml). Bakers yeast (5 g was added and the mixture stirred vigorously for 18h at room temperature. The yeast was removed by centrifugation and the aqueous layer acidified to pH 4 then extracted with ethyl acetate (4 × 25 ml). The combined extracts were dried (magnesium sulphate), evaporated to an oil (0.076 g) which was dissolved in methanol (1 ml) and treated with sodium bicarbonate (0.013 g) in water (1 ml). Evaporation to dryness gave sodium pseudomate D (0.076 g, 50%):

i.r spectrum
$\nu_{max}$ (nujol) 3400 (broad), 1710, 1647 and 1558 (broad $cm^{-1}$;

U.V. spectrum
$\lambda_{max}$ (EtOH) 223 nm ($\varepsilon_m$14,404);
$^1H$ nmr and $^{13}C$ nmr
δH and δC indicated that the product consisted of ≥80% *trans* (≤20% *cis*) of the C4', 5' double bond.

Major isomer:
$^1H$ Nmr
δH ($CD_3OD$) 0.95 (3H, d, $CH_3-17$), 1.20 (3H, d, $CH_3-14$), 1.42 (2H, m, $CH_2-7'$), 1.65 (2H, m, $CH_2-8'$), 2.01 (2H, m, $CH_2-6'$), 2.18 (3H, d, $CH_3-15$), 2.23 (4H, m, $CH_2-2'$; $CH_2-3'$), 4.06 (2H, t, $CH_2-9'$), 5.46 (2H, m, CH−4'; CH−5'), 5.74 (1H, s, H−2);

$^{13}C$ nmr
δC ($CD_3OD$) 181.8 (C1'), 168.4 (C1), 158.8 (C3), 131.5 (C5'), 130.9 (C4'), 118.3 (C2), 76.3 (C5), 71.6 (C13), 70.8 (C7), 70.0 (C6), 66.3 (C16), 64.7 (C9'), 61.5 (C11), 56.8 (C10), 43.9 (C12), 43.8 (C4), 41.5 (C8), 38.8 (C2'), 33.1, 33.0 (C6', 9), 30.5 (C7'), 29.3 (C8'), 27.0 (C3'), 20.5 (C14), 19.4 (C15), 12.3 (C17);
Microanalysis

| | | |
|---|---|---|
| (Found: | C, 59.7; | H, 7.8 |
| $C_{26}H_{41}O_9Na$ requires | C, 60.0; | H, 7.9%. |

Biological Data
a) Mycloplasma

The activity of the compounds of the Examples against various mycoplasmal organisms was assayed *in vitro* in Friis broth solidified with 0.9% agarose and innoculated with $10^3$ to $10^5$ C.F.U. The minimum inhibitory concentrations (MIC's) were determined after incubation for 6 days at 37°C and are shown in Table 1.

b) Veterinary Bacteria

The activity of the compounds of the Examples against various veterinarily important bacteria, was assayed *in vitro* using two-fold serial dilutions in Diagnostic Sensitivity Test Agar inoculated with $10^4$ organisms. The MIC's were determined after incubation for 18 hours at 37°C and are shown in Table 2.

c) Human Bacteria

The activity of the compounds of the Examples against various bacteria which are important in diseases of humans, was assayed *in vitro* using serial dilutions in nutrient agar with 5% chocolated horse blood. The MIC's were determined after incubation for 18 hours at 37°C and are shown in Table 3.

Table 1
MIC's (µg/ml) against Mycloplasma

| Organism | Compound of Example No. 1 | Compound of Example No. 2 |
|---|---|---|
| M. suipneumoniae NB12 | 1.0 | 5.0 |
| M. suipneumoniae JF 435 | 5.0 | 5.0 |
| M. suipneumoniae HK (2) | 5.0 | 5.0 |
| M. suipneumoniae STR. 11 | 1.0 | 2.5 |
| M. suipneumoniae J2206/183[b] | 2.5 | 5.0 |
| M. suipneumoniae MS 16 | 2.5 | 2.5 |
| M. suipneumoniae PW/C/210 | 1.0 | 2.5 |
| M. suipneumaniae LABER | 2.5 | 2.5 |
| M. suipneumoniae UCD 1 | 5.0 | 5.0 |
| M. suipneumoniae TAM 6N | 5.0 | 10 |
| M. suipneumoniae ATCC 25095 | 1.0 | 2.5 |
| M. suipneumoniae NCTC 10110 | 5.0 | 5.0 |
| M. hyorhinis ATCC 23234 | 1.0 | 1.0 |
| M. hyorhinis ATCC 25021 | 1.0 | 1.0 |
| M. hyosynoviae ATCC 25591 | 0.5 | 0.5 |
| M. bovis NCTC 10131 | 0.05 | 0.05 |
| M. bovigenitalium ATCC 14173 | 0.1 | 0.1 |
| M. dispar NCTC 10125 | 2.5 | 1.0 |
| M. gallisepticum S6 | >10 | >10 |
| M. pneumoniae ATCC 15492 | NG | >10 |

NG — Organism failed to grow in test
NT — Not tested

Table 2
MIC's (µg/ml) against Veterinary Bacteria

| Organism | Compound of Example No. | |
|---|---|---|
| | 1 | 2 |
| E. coli NCTC 10418 | >20 | 40 |
| E. coli E1 | >20 | 40 |
| S. dublin S7 | >20 | 80 |
| S. typhimurium S18 | >20 | 80 |
| Bord. bronchiseptica B08 | 10 | 40 |
| Bord. bronchiseptica B09 | 5 | 10 |
| Past. multocida PA1 | 0.156 | 10 |
| Past. multocida PA2 | <0.019 | 2.5 |
| Past. haemolytica PA 5 | 2.5 | 1.25 |
| Erusipelothrix rhusiopathiae NCTC 8163 | 10 | 20 |
| Corynebacterium pyogenes CY1 | NT | 80 |
| Staph. aureus B4 (pen. resistant) | 0.312 | 80 |
| Staph. Aureus (pen. sens) | 0.312 | 0.625 |
| Staph. aureus Oxford | 0.312 | 0.625 |
| Strep. suis (group D) SPS 11 | 1.25 | 0.625 |
| Strep. uberis SPU1 | 0.039 | 5.0 |
| Strep. dysgalactiae SPD1 | 0.156 | 0.156 |
| Strep. agalactiae SPA1 | 0.625 | 0.312 |
| B. subtilis ATCC 6633 | NG | NT |

NG — organism failed to grow in test
NT — not tested

Table 3
MIC's (µg/ml) against Human Bacteria

| Organism | Compound of Example No. | |
|---|---|---|
| | | 2 |
| E. coli NCTC 10418 | >25 | >100 |
| E. coli Ess | 1.0 | 1.0 |
| P. mirabilis 889 | >25 | >100 |
| K. aerogenes A | >25 | >100 |
| Ps. aeruginosa NCTC 10662 | >25 | >100 |
| Pasteurella multocida 1633 | 0.25 | 0.5 |
| Haemophilus influenzae Q1 | 0.25 | 0.5 |
| Haemophilus influenzae Wy21 | — | 0.1 |
| Neisseria catarrhalis 1502 | 0.5 | 0.5 |
| Bacillus subtilis 6633 | 1.0 | 0.5 |
| Corynebacterium xerosis 9755 | >25 | >100 |
| Sarcina lutea 8340 | >25 | >100 |
| Staph. aureus Oxford | 1.0 | 0.5 |
| Staph. aureus Russell | 1.0 | 2.5 |
| Staph. aureus W2827 | 1.0 | 1.0 |
| Strep. faecalis I | >25 | >100 |
| Strep. pyrogenes R80/421—A | 0.5 | NT |
| Strep. pyrogenes B2788 | 0.5 | 2.5 |
| Strep. pyogenes 64/848—C | 0.25 | NT |
| Strep. pneumoniae CN33 | 0.25 | 2.5 |

NT — not tested

**Claims**

1. A process for producing pseudomonic CID D of formula (III)

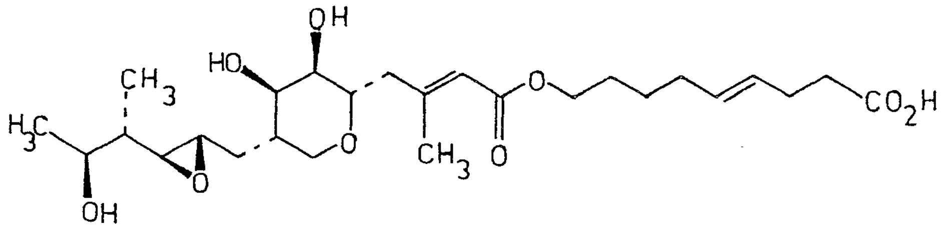

or a salt or ester thereof, which process comprises culturing an organism of *Pseudomonas fluorescens* capable of producing Pseudomonic acid D, recovering from the culture medium pseudomonic acid D of formula (III) and thereafter if desired forming a salt or ester thereof.

2. A process according to claim 1 wherein the organism of *Pseudomonas fluorescens* is NCIB 10586.

3. A process according to claim 1 or claim 2 which further process comprises hydrogenating the pseudomonic acid D of formula (III) as defined in claim 1 obtained to pseudomonic acid A of formula

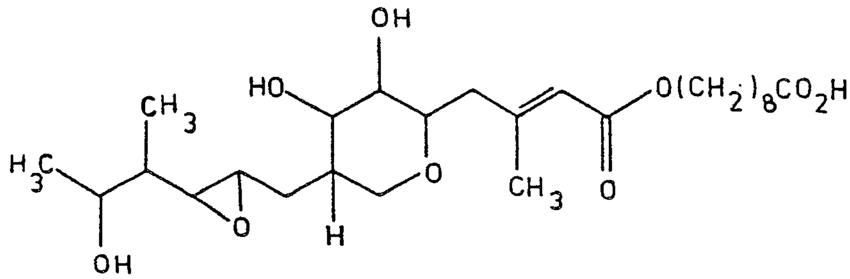

4. A process according to claim 1 or claim 2 which process further comprises hydrolysing the pseudomonic acid D of formula (III) as defined in claim 1 obtained to monic acid A of formula

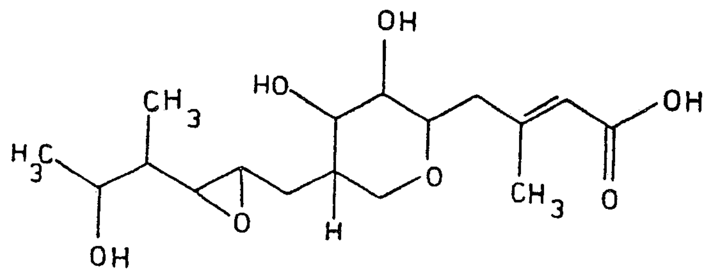

5. A process according to claim 1 wherein the pseudomonic acid D is converted to an alkali metal salt thereof.

6. A process according to claim 7 wherein the alkali metal salt is Sodium pseudomonate D.

7. A process according to claim 1 wherein pseudomonic acid D is converted to a $C_{1-6}$ alkyl ester of pseudomonic acid D.

8. A process according to claim 7 wherein the $C_{1-6}$ alkyl ester is methyl pseudomonate D.

**Patentansprüche**

1. Ein Verfahren zur Herstellung von Pseudomonsäure D der Formel (III)

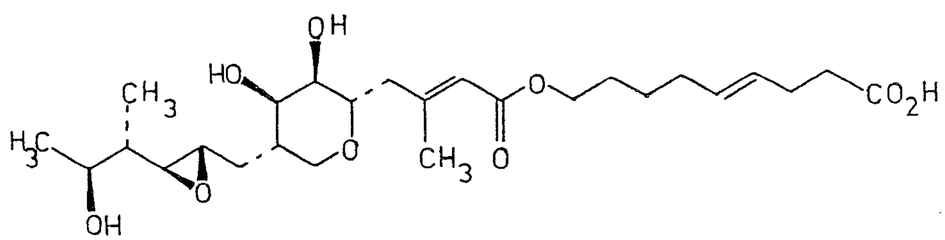

oder eines Salzes oder Esters derselben, welches Verfahren das Züchten eines Organismus der Gattung *Pseudomonas fluorescens*, der fähig ist Pseudomonsäure D zu bilden, das Gewinnen von Pseudomonsäure D der Formel (III) aus dem Kulturmedium und anschließend, falls erwünscht, das Bilden eines Salzes oder Esters derselben, unfaßt.

2. Ein Verfahren gemäß Anspruch 1, bei welchem der Organismus der Gattung *Pseudomonas fluorescens*, NCIB 10586 ist.

3. Ein Verfahren gemäß Anspruch 1 oder 2, welches Verfahren weiterhin das Hydrieren der erhaltenen Pseudomonsäure D der Formel (III), wei in Anspruch 1 definiert, zu Pseudomonsäure A der Formel

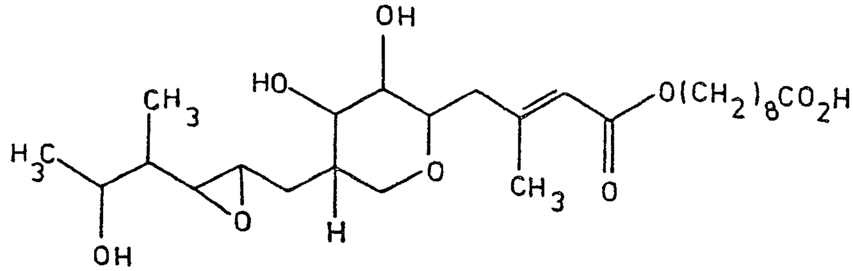

unfaßt.

4. Ein Verfahren gemäß Anspruch 1 oder Anspruch 2, welches Verfahren weiterhin das Hydrolysieren der erhaltenen Pseudomonsäure D der Formel (III), wie in Anspruch 1 definiert, zu Monsäure A der Formel

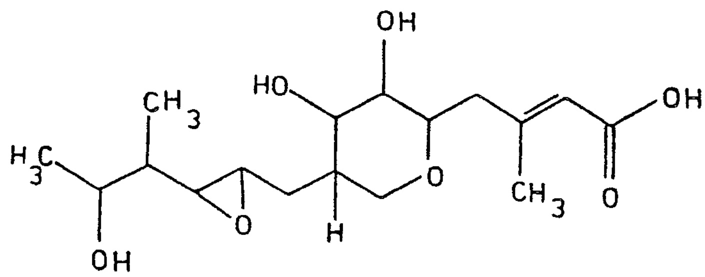

5. Ein Verfahren gemäß Anspruch 1, in welchem die *Pseudomonsäure* D in ein Alkalimetallsalz derselben umgewandelt wird.

6. Ein Verfahren gemäß Anspruch 7, in welchem das Alkalimetallsalz Natriumpseudomonat D ist.

7. Ein Verfahren gemäß Anspruch 1, in welchem Pseudomonsäure D in einen $C_{1-6}$-Alkyester von Pseudomonsäure D umgewandelt wird.

8. Ein Verfahren gemäß Anspruch 7, in welchem der $C_{1-6}$-Alkylester Methylseudomonat D ist.

**Revendications**

1. Procédé de production de l'acide pseudomonique D de formule (III)

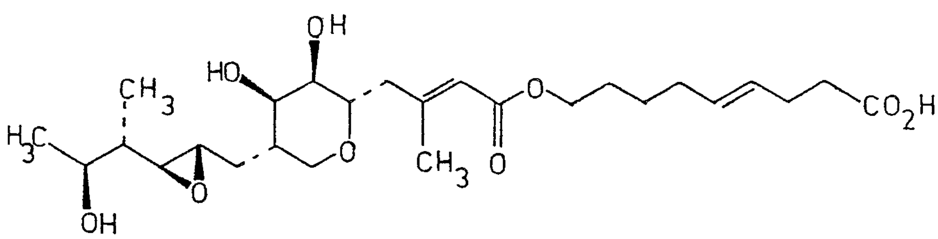

ou d'un sel ou d'un ester de celui-ci, caractérisé en ce qu'il comprend la culture d'un organisme de *Pseudomonas fluorescens* capable de produire l'acide pseudomonique D, la récupération de l'acide pseudomonique D de formule (III) à partir du milieu de culture et ensuite, éventuellement, la formation d'un sel ou d'un ester celui-ci.

2. Procédé suivant la revendication 1, caractérisé en ce que l'organisme *Pseudomonas fluorescens* est le NCIB 10586.

3. Procédé suivant la revendication 1 ou 2, qui comprend de plus l'hydrogénation de l'acide pseudomonique D. ainsi obtenu de formule (III) telle que définie dans la revendication 1, en l'acide pseudomonique A de formule

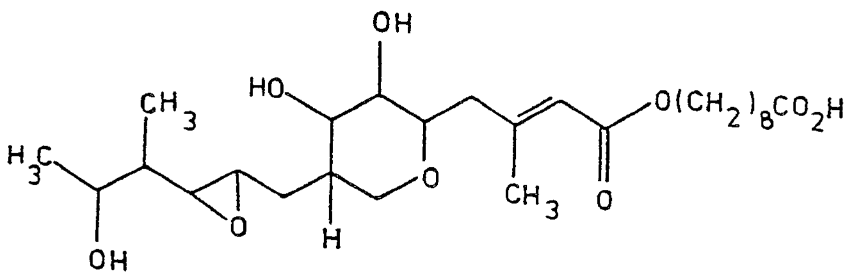

4. Procédé suivant la revendication 1 ou la revendication 2, qui comprend de plus l'hydrolyse de l'acide pseudomonique D ainsi obtenu de formule (III) telle que définie dans la revendication 1, en l'acide monique A de formule

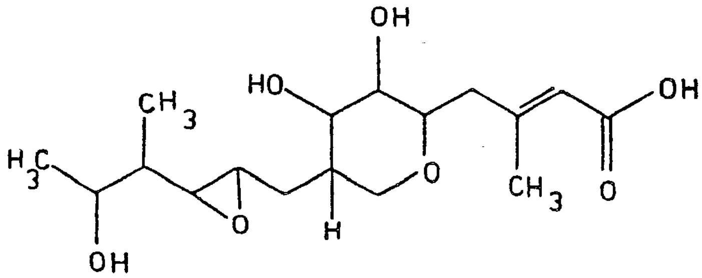

5. Procédé suivant la revendication 1, caractérisé en ce que l'acide pseudomonique D est converti en sel de métal alcalin de celui-ci.

6. Procédé suivant la revendication 5, caractérisée en ce que le sel de métal alcalin est le pseudomonate D de sodium.

7. Procédé suivant la revendication 1, caractérisé en ce que l'acide pseudomonique D est converti en un ester alcoylique en $C_{1-6}$ de l'acide pseudomonique D.

8. Procédé suivant la revendication 7, caractérisé en ce que l'ester alcoylique en $C_{1-6}$ est le pseudomonate D de méthyle.